# EUROPEAN PATENT APPLICATION

(11) **EP 2 977 752 A1**
(43) Date of publication of application: **27.01.2016**
(21) Application number: 15177871.9
(22) Date of filing: 22.07.2015
(51) Int. Cl.: G01N 27/22, G06K 19/07, H04B 5/00

(54) **SYSTEM AND METHOD FOR MEASURING MOISTURE IN A STRUCTURE**

(30) Priority: 25.07.2014 FI 20145693
(71) Applicant: Nallen Kylpyhuoneet ja Saunat Oy, 00701 Helskinki (FI)
(72) Inventor: Talvensaari, Juha, 00701 Helsinki (FI)
(74) Representative: Kolster Oy Ab

(57) **Abstract**

The invention relates to a wirelessly readable moisture sensor, a method and system for measuring moisture, software for a mobile device, and the use of a mobile phone for reading the result of a moisture measurement from a moisture sensor embedded into a structure.

## Description

### Background of the invention

The invention relates to moisture measurements and especially to measuring moisture in wet rooms of buildings.

Moisture accumulating in the structures of buildings and houses, especially residential buildings, causes significant indoor air and mould problems that are detrimental to human beings. By measuring the moisture in the structures, it is possible to solve incipient problems before the moisture in the structure has time to damage the structures and weaken the quality of indoor air. Reliable moisture measurement of structures is difficult, and two general ways of doing it are used: surface measurement without damaging the structures and measuring through a trial bore, whereby the surface material and possible damp-proofing of the space are damaged. Surface measurement does not give a reliable picture of moisture deep in the structure, so typically the only way to confirm the condition of the structures is to drill a trial bore and measure through it, which gives a reliable result, but at the same time damages a possibly completely intact and dry structure in vain. There is also a continuous need for moisture measurements in the industry.

PCT publication WO2013030430 shows a method for non-invasive moisture measurement of structures. The method is based on sensors that are installed inside the structures during building and can later be read by a wireless reader made for this purpose. The sensors can also be installed during renovation, in which case dents for embedding the sensors need to be made to the structures by drilling and/or cutting. In some corresponding systems, special mortar also needs to be used for fastening the sensors to the dents made for them. In some corresponding systems, measuring results can be stored on a server of the system vendor after making the measurements.

A problem with the arrangement described above is the large size of the sensors, which requires a lot of extra work especially when they are installed during renovation. Another problem is the reader device that is dependent on the system vendor, in which case as technology advances or the manufacturer stops making the product, it may be difficult to obtain the reader devices. In the case of parallel systems of different manufacturers, the persons measuring moisture, renovating and building need to have a large number of reader devices from different manufacturers so that they can test the operation of the sensors of each manufacturer and measure moisture values from the sensors. For private persons, the acquisition of reader devices for reading the measuring results of their home is a disproportionately large investment.

### Brief description of the invention

An object of the invention is thus to provide a method and equipment implementing the method so as to solve the aforementioned problems. The object of the invention is achieved by a method and system which are characterised by what is disclosed in the independent claims. Preferred embodiments of the invention are disclosed in the dependent claims.

The invention is based on the fact that it is possible to read wirelessly the relative moisture and temperature measured by sensors left inside the structures by using any mobile device with a suitable software and support for the wireless connection used by the sensor.

The method and system of the invention provide the advantage that they are independent of the system vendor, because it is possible to use conventional mobile devices, such as phones and tablet computers, available for consumers without any system vendor-specific auxiliary devices. The sensors are preferably made on flexible, thin circuit boards so that no dents need to be made in existing structures but the sensors can be installed in the filler or damp-proofing layer, for instance.

### Brief description of the figures

The invention will now be described in more detail in connection with the preferred embodiments and with reference to the accompanying drawings, in which:
Figure 1 shows a wet area, in which sensors according to an embodiment have been installed; and
Figure 2 shows by way of example a structural view of the positioning of a sensor in a structure, and of the measurement.

### Detailed description of the invention

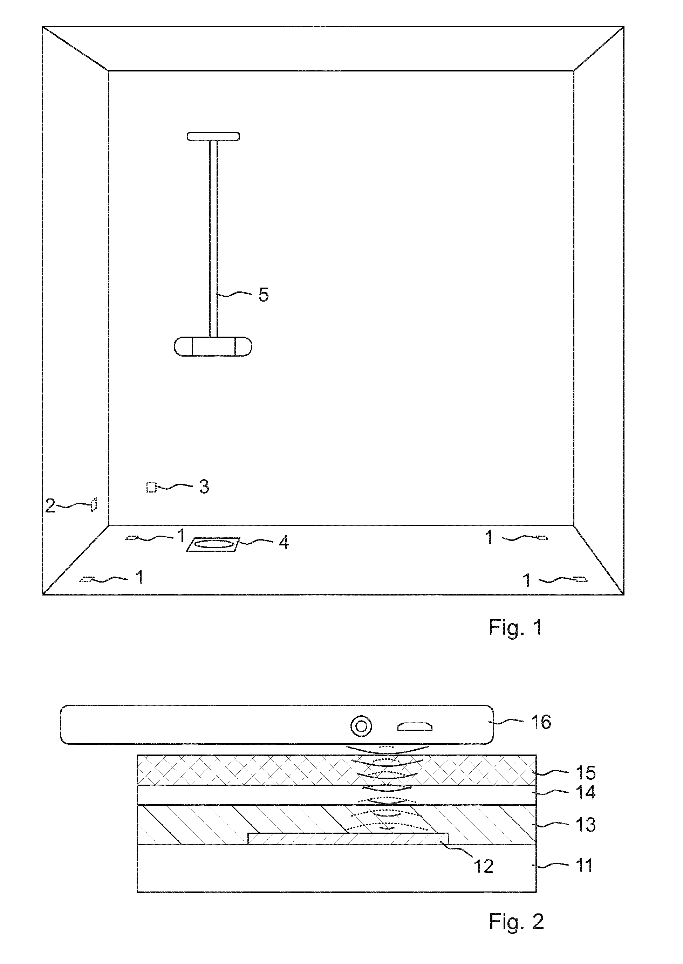

With reference to Figure 1, moisture sensors 1, 2, 3 (later sensor) of the system can be installed at a distance from the corners of the space as shown in the figure, for example. The most typical leakage points, from which moisture enters from the space to the structure, are in the vicinity of seams and corners. Especially in the vicinity of points where moisture stress is the highest, such as around a floor drain 4 and in a shower corner and in the vicinity of the shower 5 in general, it is advantageous to install a sensor to measure the temperature and/or moisture, preferably the relative moisture of the structure. It is recommended to install the sensors slightly away from the structure at risk, such as a corner or joint, because it is technically easier and reduces possible damage to the risk structure and sensor. The sensors may be installed as floor sensors 1 and wall sensors 2, 3. Preferably at least one sensor is always installed in a predetermined location regardless of the space, in which case the sensor is easy to find with a measuring device even when information on the installation sites of all sensors was not available in advance. A predetermined installation site is preferably in the area of high moisture stress, whereby just measuring it reveals if there are significant moisture problems in the structures. The predetermined location may be at a water point in the wall 20 cm from floor level, on the floor halfway along the line between the floor drain and shower, or on the floor at a distance of 25 cm from both walls of the corner closest to the shower. Depending on the end-use, other predetermined sites may be defined. In an embodiment, sensors are also installed in areas where moisture stress is very low or non-existent. Sensors installed in these areas can be used as reference points in the moisture measurement to mainly show the relative moisture of a healthy structure, with which it is good to compare the measuring results from areas of higher moisture stress. Structures typically contain moisture transmitted from outdoor air or the ground, for instance, which means that the relative moisture of the structure may vary depending on the weather conditions, seasons and indoor and outdoor temperatures. To confirm that such variations are caused by external circumstances, it is advantageous to use reference sensors.

With reference to Figure 2, a typical structure may be formed as follows, for instance: on top of a concrete slab, plaster board, plywood or some other base structure or filler layer 11, there is a wirelessly readable sensor 12 inside damp-proofing 13 in such a manner that the sensor 12 is by its bottom surface in contact with the base structure or filler layer 11 below it. On top of the damp-proofing layer 13, there is renovation mortar 14 or installation glue, to which tiles 15 or a plastic carpet, for instance, is fastened as surface material. Alternatively, the sensor 12 can also be installed in the filler layer 11, either completely inside it or against a base structure, such as concrete slab, plaster board or plywood, below it. If moisture continuously seeps into the structure behind the damp-proofing 13, as seen from the room, moisture will slowly spread to a wide area, if it cannot exit efficiently from the structure. Therefore, different practices, even other than the examples presented above, can be applied to the positioning the sensor 12 to the structure depending on the structure and its materials.

Figure 2 also shows a measuring operation, in which information and energy can be transmitted and received to and from the sensor 12 wirelessly with a measuring device 16 from the room-side against the surface material or in its vicinity. Any programmable device that supports the wireless technology used by the sensor can, in principle, be use as a reader device. Preferably, a phone is used that typically has extensive wireless interfaces and easily installable software for communication with the sensor. In an embodiment, sensors based on NFC (*Near Field Communication*) technology and a phone that supports NFC technology are used, whereby data transmission between the sensor and phone takes place by using NFC, and at the same time the necessary energy is also supplied to the sensor by means of NFC. Correspondingly, in an embodiment, it is possible to use in the sensor and reader device other wireless connections and technologies, such as Bluetooth, RFID, and WLAN, used in mobile devices available for consumers either independently or together with some other wireless technology. In a preferred embodiment, the reader device is a phone that is capable of executing software for the purpose of communicating with the sensor.

In the following, the operational structure of the sensor is disclosed without going into specific technical details. The sensor is based on combinations of state-of-the-art technical solutions, so it is obvious to a person skilled in the art that each function can be technically implemented in several alternative ways and it is not necessary to describe the individual known technologies in more detail herein.

The sensor comprises a circuit that comprises at least an antenna and one sensor for making measurement and for transforming the measuring results into electronic format. The sensor is preferably made into a flexible circuit that is a few millimetres, preferably less than 3 mm and most preferably less than 2 mm, in thickness. For a thin sensor, it is not typically necessary to make dents into the structures but the sensor can be installed in the appropriate layers of the structure.

The sensor 1, 2, 3, 12 preferably comprises an antenna for transmitting and receiving information and for receiving energy so that the sensor receives the energy it needs through the antenna from the reader device. In an embodiment, the antenna operates on several frequency bands, of which at least one is used to transmit energy from the reader device to the sensor and at least one other frequency band is used for transmitting information between the sensor and reader device. In an embodiment, the sensor comprises several antennas, of which at least one is used to transmit energy from the reader device to the sensor and at least one other antenna is used for transmitting information between the sensor and reader device. There are numerous technical solutions for wireless energy transmission in the present embodiment. In practice, the distance between the antenna of the reader device and the antenna varies from less than a centimetre to a few centimetres, and the necessary power is typically well under one wat in size. Patent publication US20100190436 discloses a solution for this type of short-range power and data transmission over radio waves.

The antenna of the sensor is preferably in connection with a microcircuit, in which case the energy and information received by the antenna are transmitted to the microcircuit. The microcircuit is adapted to receive and transmit information via the antenna to and from the reader device. The microcircuit handles the information received by the sensor antenna and processes it as necessary. The sensor also comprises a moisture sensor for measuring the relative moisture of the structure or the space with which it is physically in contact. The moisture sensor transforms the measured relative moisture into electronic information for the microcircuit and to be transmitted on to the reader device. In an embodiment, the sensor also comprises a temperature sensor for measuring the temperature and transforming it into electronic information for the microcircuit and to be transmitted on to the reader device. In an embodiment, the sensor 12 also comprises other sensors for measuring quantities related to the structures and for transforming them into electronic information for the microcircuit and to be transmitted on to the reader device. In an embodiment, two or more of the measurements are made with one multi-purpose sensor. In an embodiment, one or more sensors are connected by conductors to the sensor and its microcircuit so that the sensors can be positioned more freely and it is possible to drill a small hole into the structure for them, in which case the entire sensor need not be fitted into the hole.

In an embodiment, the sensor comprises a non-volatile memory either as part of said microcircuit or as a separate memory that is connected to the microcircuit. The microcircuit may store into the memory, read from the memory, and transmit information between the memory and reader device via the antenna. In an embodiment, the measuring results read from one or more sensors are stored into the memory. In connection with the measuring results, the measuring time, which may be received from the reader device or obtained from the clock of the microcircuit or a clock circuit attached thereto, is preferably stored. Of the stored measuring results, it is possible to examine on the reader device measuring results over the entire service life of the sensor and easily detect possible deviations and periodical variations due to the weather or season, for instance. In an embodiment, information on the structure, in which the sensor is installed, can be stored into the memory. The information on the structure may comprise material information on the structure, order of materials in the structure, material thicknesses, permitted relative moisture variation ranges in the materials, highest permitted relative moisture in the materials, general information on the structural materials and/or other information related to the structure. Information related to the structure may be stored into the memory before the sensor is installed, during the installation and/or after it by using the reader device, for example. Information related to the structure may be transmitted from the memory to the reader device, on which the information may be examined. In an embodiment, instructions related to the system may be stored into the memory. In an embodiment, the instructions related to the system comprise information on the system, the sensors and/or their positioning, the location of at least one other sensor, general information on measuring moisture with the system and/or other instructions related to the system. Information related to the system may be stored into the memory before the sensor is installed, during the installation and/or after it by using the reader device, for example. Information related to the structure may be transmitted from the memory to the reader device, on which the information may be examined. In an embodiment, it is possible to store into the memory with the reader any information, such as notes, comments, instructions, or measuring results from other sensors. Preferably, all information in the memory of the sensor can be read and processed with the reader device via the antenna and microcircuit. The memory in the sensor significantly adds to the independence of the system, because the information stored into the memory can be read with any suitable reader device at any time, and no information is lost when the reader device breaks or is changed.

In an embodiment, the sensor comprises an energy storage, such as a capacitor or low-capacity battery, to which energy received by the antenna can be temporarily stored and from which the stored energy can be used for the functions of the sensor in case of a higher energy demand or as energy supply decreases or temporarily stops.

The system according to the invention uses as the reader device any existing mobile devices, into which software suitable for reading the sensors can be installed and which have the necessary wireless connection, with which information can be transmitted between the sensor and reader device and the energy required by the sensor can be transmitted from the reader device to the sensor during use. Technology advances quickly in this field and, therefore, it is not necessary to present a comprehensive list of appropriate devices available at the time this patent application is drafted. At the time this application is drafted, a large number of the so-called smart phones functioning in mobile networks and tablet computers are sufficiently equipped for installing software suitable for reading sensors so the phone or table computer can be used as the reader device of the above-mentioned sensors. Programming the necessary software, in practice application (*app*, *applet*), is a routine task for a person skilled in the art, when a desired entity is formed of the above properties and functions and the details of data transmission between the sensor and reader device are defined. At its simplest, a user interface is required to display the information received from the sensor, if the sensor is configured in such a manner that, after starting up by means of electric current received from the antenna, it measures, without a separate command, with its sensors the relative moisture and temperature, for instance, and transmits them via the antenna to the reader device. However, the software of the reader device is preferably capable of reading the sensor memory and writing to it, of transmitting and receiving information with the sensor, and of processing, storing and displaying information received from the sensor in a versatile manner on the display of the reader device. In an embodiment, information, such as measuring results, read from the sensor can be stored via a mobile network or WLAN to a network service, from which it can be later read using the software of the reader device. For the examples of reader devices presented herein, good distribution channels already exist for third-party software, through which the software is easily and quickly available practically to anybody's phone, after which the phone functions as a reader device without any auxiliary devices by using one or more in-built wireless connections in the phone for the purpose of transmitting information or energy between the phone and sensor.

A first aspect according to the invention is a wirelessly readable moisture sensor that comprises at least one sensor for measuring relative moisture and temperature and transforming them into electronic information, and antenna for transmitting the information to a reader device and for receiving information and energy from the reader device, as well as a microcircuit at least for processing the received information that is to be transmitted. The moisture sensor is characterised in that it also comprises a permanent memory in connection with the microcircuit, into which information can be stored and from which information stored therein can be read with the reader device. In an embodiment, the moisture sensor is made into a flexible circuit. In an embodiment, at least one sensor protrudes from said flexible circuit. In an embodiment, said at least one protruding sensor is separate from said flexible circuit, and said at least one protruding sensor is connected by conductors to said flexible circuit. In an embodiment, it is possible to store into the memory of the moisture sensor and to read from it by the reader device at least one of the following pieces of information: earlier measuring result, sensor location information, structure information, material information, and instructions for use.

Another aspect according to the invention is a method for measuring moisture. In the method, a reader device is brought close to a moisture sensor, a wireless connection is established between the reader device and moisture sensor for transmitting information and energy from the reader device to the moisture sensor and for receiving information from the moisture sensor to the reader device, a measurement of at least the relative moisture is made with the moisture sensor and the measuring result is transmitted over the wireless connection, and the measuring result transmitted by the moisture sensor is received and displayed on the reader device. The method is characterised in that the reader device is a mobile device that is independent of said moisture sensors and said wireless connection between the reader device and moisture sensor is established using the in-built connection means of the mobile device, the mobile device executing software for displaying and transmitting information with the moisture sensor and for transmitting energy wirelessly from the mobile device to the moisture sensor.

In an embodiment of the method, said mobile device that is independent of the moisture sensors is a mobile phone, preferably smart phone, whose wireless connections are compatible with the moisture sensor. In an embodiment, the measuring result from the measurement made by the moisture sensor is also stored into a permanent memory of the moisture sensor, which is readable by the reader device. In an embodiment, the sensor is embedded into a structure of the building and the reader device is brought close to the moisture sensor without damaging said structures. In an embodiment, not only the relative moisture but also the temperature is measured.

A third aspect according to the invention is software for a mobile device. The software according to the aspect is characterised in that executing the software in the mobile device in the vicinity of a moisture sensor according to the first aspect according to the invention makes the mobile device execute the steps of the method, comprising establishing a wireless connection between the reader device and moisture sensor for transmitting information and energy from the reader device to the moisture sensor and for receiving information from the moisture sensor to the reader device, receiving and displaying on the reader device a measuring result transmitted by the moisture sensor.

In an embodiment, the mobile device of the software according to the third aspect of the invention or of the method according to the second aspect of the invention is a mobile phone or smart phone.

A fourth aspect according to the invention is a system for measuring moisture. The system is characterised in that it comprises the moisture sensor according to the first aspect of the invention and the software according to the third aspect of the invention.

A fifth aspect according to the invention is the use of a mobile phone, preferably smart phone, for reading the result of a moisture measurement wirelessly from a moisture sensor embedded into a structure. In an embodiment, the moisture sensor is the moisture sensor in accordance with the first aspect of the invention. In an embodiment, the result of the moisture measurement is read wirelessly using in-built connection means of the mobile phone.

It is apparent to a person skilled in the art that as technology advances, the basic idea of the invention may be implemented in many different ways. The invention and its embodiments are thus not restricted to the above-described examples but may vary within the scope of the claims.

## Claims

1. A wirelessly readable moisture sensor (12) that comprises at least one sensor for measuring relative moisture and temperature and for transforming them into electronic information, an antenna for transmitting the information to a phone (16) and for receiving information and energy from the phone (16), and a microcircuit for processing at least the received information and information to be transmitted, **characterised in that** the moisture sensor (12) also comprises in connection with the microcircuit a permanent memory, into which information can be stored and from which stored information can be read with the phone, **characterised in that** data transmission between the moisture sensor (12) and phone (16) takes place by NFC technology, and the energy required by the sensor is supplied by NFC technology.

2. A moisture sensor as claimed in claim 1, **characterised in that** it is made into a flexible circuit.

3. A moisture sensor as claimed in claim 1 or 2, **characterised in that** at least one of the following pieces of information is storable into said memory and readable therefrom by the phone: earlier measuring result, location information of the sensor, structure information, material information, and instructions for use.

4. A method for measuring moisture, comprising the steps of:
- bringing a reader device (16) close to the moisture sensor (12),
- establishing a wireless connection by NFC technology between the reader device and moisture sensor for transmitting information and energy from the reader device to the moisture sensor and for receiving information from the moisture sensor to the reader device,
- measuring at least the relative moisture with the moisture sensor and transmitting the measuring result over the wireless connection, and
- receiving the measuring result transmitted by the moisture sensor and displaying it on the reader device,
wherein the method is **characterised in that** the reader device (16) is a phone that is independent of said moisture sensors and said wireless connection between the reader device and moisture sensor is established using the in-built connection means of the phone, the phone executing software for displaying and transmitting information with the moisture sensor and for transmitting energy wirelessly from the phone to the moisture sensor.

5. A method as claimed in claim 4, **characterised by** also storing the measuring result from the measurement made by the moisture sensor into a permanent memory of the moisture sensor, which is readable by said reader device.

6. A method as claimed in claim 4 or 5, **characterised in that** the sensor is embedded into a structure (11, 13, 14, 15) of a building and the reader device (16) is brought close to the moisture sensor (12) without damaging said structures.

7. Software for a phone, **characterised in that** running the software in the phone in the vicinity of a moisture sensor according to claim 1 makes the phone execute the steps of the method comprising
- establishing a wireless connection by NFC technology between the phone and moisture sensor for transmitting information and energy from the phone to the moisture sensor and for receiving information from the moisture sensor to the phone, and
- receiving a measuring result transmitted by the moisture sensor to the phone and displaying it on the phone.

8. A system for measuring moisture, **characterised in that** it comprises a moisture sensor according to claim 1 and software according to claim 7.

9. The use of a mobile phone for reading the result of a moisture measurement wirelessly using NFC technology from a moisture sensor embedded into a structure.
